# FASCICULE DE BREVET EUROPEEN

(11) **EP 0 772 473 B1**
(45) Date de publication et mention de la délivrance du brevet: **25.11.1998**
(21) Numéro de dépôt: 95925888.0
(22) Date de dépôt: 13.07.1995
(51) Int. Cl.: A61M 35/00, A45D 34/04

(54) **NOUVEAU DISPOSITIF DE BADIGEONNAGE**
EINE EINRICHTUNG ZUM AUFTRAGEN
NOVEL SKIN SWAB DEVICE

(30) Priorité: 22.07.1994 FR 9409083
(43) Date de publication de la demande: 14.05.1997
(73) Titulaire: Laboratoires Demic, 91120 Palaiseau (FR)
(72) Inventeur: CANDELA, Michel, F-91370 Verrières-le-Buisson (FR)
(74) Mandataire: Burtin, Jean-François
(86) Numéro de dépôt international: FR9500948
(87) Numéro de publication internationale: WO9603177

(56) Documents cités:
- EP-A- 0 081 816
- EP-A- 0 232 596
- EP-A- 0 375 579
- DE-A- 1 940 235
- GB-A- 1 479 480
- US-A- 4 887 994
- US-A- 5 122 158

## Description

La présente invention concerne un dispositif de badigeonnage destiné à l'application sur la peau, par frottement, de produits de désinfection, d'aseptisation ou de traitement cutané, en particulier lors d'interventions chirurgicales ou de soins cutanés, et/ou transcutanés, chez l'homme ou chez l'animal.

Le dispositif peut être présenté stérile ou non stérile, selon les nécessités thérapeutiques. En fait, l'utilisation la plus fréquente devrait être la forme stérile à usage unique.

La pratique actuelle des badigeonnages chirurgicaux, appliquée depuis fort longtemps, consiste en des badigeonnages à l'aide de compresses tenues par une pince. Cette opération comporte un certain nombre de gestes nécessités par l'obligation de respecter des normes aseptiques afin d'assurer la sécurité des patients :
- stérilisation de la pince et des compresses avant usage
- fixation de la compresse sur la pince
- mise à disposition du liquide désinfectant dans un récipient stérile
- trempage fréquent de la compresse pour un badigeonnage efficace.

Tous ces gestes se traduisent nécessairement par :
- une perte de temps pour le personnel soignant
- un gaspillage du produit de désinfection ou de traitement
- un processus d'assurance de qualité obligatoire (certitude de la stérilisation, comptage des compresses, ....)
- un temps de badigeonnage long dû à la faible surface des compresses
- une salissure inévitable des surfaces entourant le patient, les produits utilisés étant souvent colorés.

Pour résoudre ces problèmes, des systèmes de badigeonnage par pulvérisation ou roulement ont été proposés, mais leurs avantages sur le plan bactériologique et clinique par rapport aux systèmes par frottement, n'ont pas été démontrés de façon significative.

En outre, la conception de tels systèmes de badigeonnage par pulvérisation peut entraîner des coûts de production élevés, donc une acceptation plus difficile par le personnel hospitalier dans le domaine des produits à usage unique.

Pour pallier ces inconvénients, le système de l'invention rend possible un étalement du produit sur la peau, et un badigeonnage pratique, rapide, sûr et économique.

Ainsi, le dispositif selon l'invention telle que définie dans la revendication 1 se présente sous forme d'un ensemble formé de trois parties :
- un réservoir de produit composé d'un flacon en plastique souple contenant le produit de désinfection ou de traitement, et pouvant être pressé dans la main, pour expulser le produit.
- un corps intermédiaire se vissant sur le flacon du réservoir, et ayant à l'intérieur un canal amenant le produit jusqu'à une grille percée de trous.
- un applicateur en matériau spongieux, arrondi, dont l'embout est enserré dans le corps intermédiaire et reçoit le produit par les trous de la grille. L'applicateur en mousse, pressé sur la peau, diffuse le produit par frottement du dispositif sur la surface cutanée.

Les différents matériaux utilisés pour réaliser l'ustensile doivent être compatibles avec les exigences sanitaires.

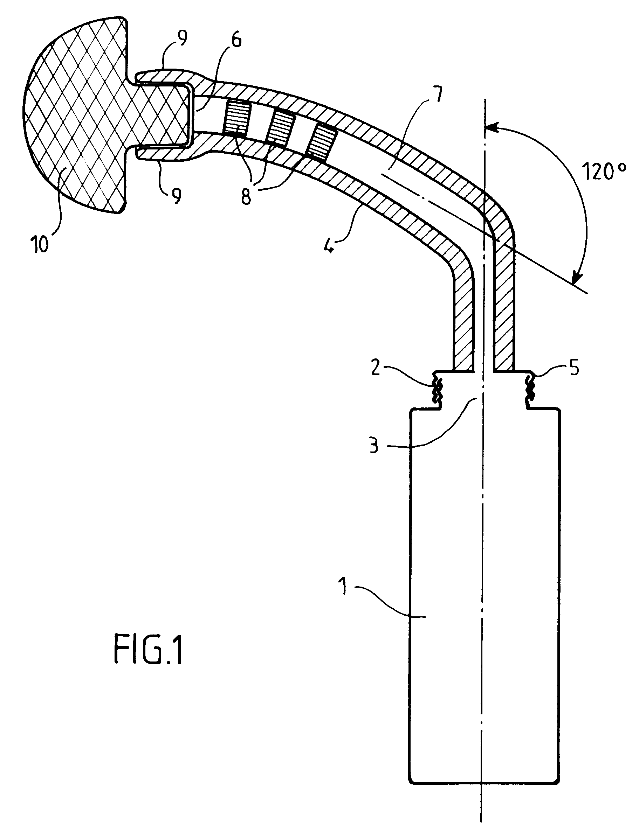

La figure 1 représente, en coupe, le dispositif selon l'invention. En référence à ce dessin, le dispositif comporte :
- le réservoir (1) constitué d'un flacon souple que l'on remplit au moment de l'utilisation (ou qui est préalablement rempli). Le flacon est pourvu d'un pas de vis (2) coopérant avec celui de la partie intermédiaire. Le corps du flacon est suffisamment élastique pour que l'on puisse exercer une pression simplement avec la main de l'utilisateur. L'orifice d'ouverture du flacon (3) est d'un diamètre suffisant pour assurer un remplissage facile. La capacité du flacon est adaptée à l'utilisation qui est projetée. A titre d'exemple, la capacité peut varier de 50 à 300 cm³.
- le corps intermédiaire (4), de forme recourbée (angle d'environ 120° par rapport au manche), afin d'épouser parfaitement, lors du badigeonnage, les courbes cutanées sans gêner la main de l'utilisateur, est constitué :
   ⇒ à une extrémité, d'un pas de vis (5) sur lequel on visse le flacon
   ⇒ à l'autre extrémité, d'une grille (6) assurant la diffusion du produit dans la partie en matériau spongieux. Les trous de la grille sont d'un diamètre suffisant pour que le produit puisse diffuser facilement, quelle que soit la viscosité du véhicule.
   ⇒ au milieu, d'une partie canalaire (7) constituant le trajet du produit. Cette partie canalaire comporte en son sein un jeu de chicanes (8) de tailles différentes afin de permettre une diffusion homogène du produit sur toute la largeur de la grille sans risque d'excès ou de projection
   ⇒ d'un dispositif d'enserrage mécanique de l'extrémité en matériau spongieux (9).
   ⇒ l'applicateur en matériau spongieux (10) est de forme arrondie afin de passer au mieux sur les parties cutanées courbes. La surface de délivrance de l'applicateur en tissu spongieux est suffisamment grande pour recouvrir une surface corporelle importante. A titre d'exemple, les dimensions du matériau spongieux peuvent être de l'ordre de 10 à 12 cm de largeur et de 3 à 5 cm de diamètre.

Le matériau spongieux dont est formé l'applicateur est un matériau inerte imputrescible alvéolé comme, par exemple, une 〈〈 mousse de polyuréthanne 〉〉.

Selon une variante non illustrée, le réservoir et le corps intermédiaire peuvent être d'une seule pièce, l'orifice de remplissage se situant à l'extrémité inférieure du réservoir et étant constitué d'un bouchon à vis ou à clip.

Le dispositif selon l'invention, représente donc une amélioration pour le badigeonnage cutané du fait de son côté pratique, rapide, sûr et économique.

## Revendications

1. Dispositif de badigeonnage formé d'un réservoir souple, d'un corps intermédiaire vissé sur le réservoir et d'un applicateur disposé à son extrémité, caractérisé en ce que ce dispositif est constitué d'un flacon souple (1) formant le manchon et pourvu d'un orifice de remplissage comportant un col fileté (3) sur lequel un embout (5) est monté en se serrant sur ledit filetage par l'intermédiaire d'un pas de vis (2), ledit embout étant traversé par un corps intermédiaire (4) de distribution qui présente un rayon de courbure d'environ 120° par rapport au manche et qui possède à la partie interne un canal à l'intérieur duquel on a disposé une série de chicanes, et à l'extrémité de laquelle est disposé dans un logement un matériau spongieux de surface arrondie débordant largement sur l'extrémité de la tubulure.

2. Dispositif de badigeonnage selon la revendication 1, dans lequel le flacon souple (1) peut être enlevé par dévissage lorsqu'il est vide.

3. Dispositif de badigeonnage selon la revendication 1, dans lequel la tubulure de distribution se termine par une grille (6) qui sert de base au logement dans lequel est inséré le matériau spongieux terminal (10), qui vient se loger par compression dans un logement de section rectangulaire disposé à l'extrémité du corps intermédiaire (4) de distribution.

4. Dispositif de badigeonnage selon l'une des revendications 1 à 3, dans lequel le matériau spongieux terminal (10) présente, en surface, vers l'extérieur, une forme en éventail et vers l'intérieur possède une partie plus étranglée de section prismatique.

5. Dispositif de badigeonnage selon la revendication 1, dans lequel le flacon (1) de remplissage est rempli du produit à badigeonner.

## Claims

1. Swabbing device made of a flexible container, an intermediate body threaded on the container and an applicator arranged at its end, characterized in that this device is constituted of a flexible bottle (1) forming a mantle and provided with a filling opening including a threaded neck (3) on which a nozzle (5) is mounted while squeezing up against the said thread through a threading (2), said nozzle being gone through with an intermediate distribution body (4) which shows a curvature radius of about 120° against the handle and which possesses in the internal part, a channel at the internal part of which a series of zick-zacks is set out and at the end thereof a spongy material with a rounded surface, broadly extending on the end of the tubing, is disposed in a housing.

2. Swabbing device according to claim 1 wherein the flexible bottle (1) may be removed by unscrewing it when empty.

3. Swabbing device according to claim 1 wherein the distribution tubing is ended by a grid (6) which is used as a bottom for the housing in which the terminal spongy material (10) is inserted, which goes to lodge by compression in a housing of rectangular shape disposed at the end of the intermediate distribution body.

4. Swabbing device according to one of the claims 1 to 3 wherein the end of the spongy material shows at its surface, outwards, a fan-shaped material and inwardly shows a more strangled part of prismatic section.

5. Swabbing device according to claim 1 wherein the filling bottle (1) is filled up with the product to be swabbed.

## Patentansprüche

1. Vorrichtung zum Bepinseln die aus eine weichen Behälter, eine zwischenliegende Boden das auf dem Behälter festgeschraubt ist und eine Applikator der an die Ende des Behälters angelegt ist, besteht,
dadurch Gekennzeichnet daß diese Vorrichtung aus einem weichen Flaschen (1) das die Büchse bildet und das mit einer eingewindeschnittene Hals (3) umfassende Abfullungs offnung, ausgerüstet ist, an dem eine Ansatz (5) der beim Drücken auf der genannte Windung über einem Schraubengewinde (2) sich drängt, daß der genannte Ansatz durch das zwischenliegende Boden fur die Verteilung durchgegangen wird, das eine Durchbiegungsradius von etwa 120° - bezogen auf dem Stiel - zeigt und das in der inneren Teil eine Röhre aufweist, innerhalb der eine Reihe von Zickzackwege angelegt sind, und an die Ende deren, in einem Gehaüse ein schwammartiges Material mit gerundete Flache angeordnet ist, das weitgehend an die Ende des Rohransatzes übersteht.

2. Vorrichtung zum Bepinseln nach Anspruch 1 worin das weiches Fläschen (1) durch Abschrauben entfernt werden kann wenn es leer ist.

3. Vorrichtung zum Bepinseln nach Anspruch 1, worin die Verteilungsansatz mit einem Gitter (6) beendet ist das als Boden fur die Gehäuse gebraucht wird, wobei das endliches schwammartiges Material eingearbeitet ist das durch Drücken in einem viereckigenquerschnitt Gehäuse an die Ende des zwischenliegenden Boden für die Verteilung angeordnet ist.

4. Vorrichtung zum Bepinseln nach einer der Ansprüche 1 bis 3, worin das endlische schwammartiges Material (10) flächig nach außen eine fächer artige Form und nach innen eine engere Teil von prismatischen Querschnitt aufweist.

5. Vorrichtung zum Bepinseln nach Anspruch 1, worin das Abfüllungsflaschen (1) mit dem zum Bepinseln Produkt, befüllt ist.
